# EUROPEAN PATENT APPLICATION

(11) **EP 4 659 664 A1**
(43) Date of publication of application: **10.12.2025**
(21) Application number: 24425024.7
(22) Date of filing: 07.06.2024
(51) Int. Cl.: A61B 5/024, A61B 5/25, A61B 5/291, A61B 5/318, A61B 5/00

(54) **HEAD-MOUNTABLE DEVICE AND ECG DETECTION SYSTEM FOR DETECTING AN ELECTROCARDIOGRAM SIGNAL COMPRISING SAID HEAD-MOUNTABLE DEVICE**

(71) Applicant: Luxottica S.r.l., 32021 Agordo (Belluno) (IT)
(72) Inventor: Trojaniello, Diana, 32021 Agordo (BL) (IT); Ongarello, Tommaso, 32021 Agordo (BL) (IT); Mainardi, Luca, 20133 Milano (MI) (IT); Cerveri, Pietro, 20133 Milano (MI) (IT)
(74) Representative: Biallo, Dario

(57) **Abstract**

Head-mountable device (100) comprising:
- a supporting structure (110) configured to be mounted on a head of a user;
- at least one first electrode (210) arranged on the supporting structure (110) so as to be in contact with the head of a user when such a user wears the head-mountable device (100) in order to detect a first electrical potential;
- at least one second electrode (220) arranged on the supporting structure (110) so as to not be continuously in contact with a body part of a user when such a user wears the head-mountable device (100) in order to detect a second electrical potential on demand; wherein said head-mountable device (100) is configured to be connected to an electronic unit (230) configured to collect the electric potentials detected by the at least one first electrode (210) and the at least one second electrode (220) and to determine the electrocardiographic signal on the basis of the difference of said detected electric potentials.

## Description

The present invention refers to a head-mountable device and to an ECG detection system for detecting an electrocardiogram (ECG) signal comprising such a head-mountable device.

An electrocardiogram (ECG) is a non-invasive medical test that records the electrical activity of the heart over a while. It is typically represented by a signal graph, known as an electrocardiogram tracing, which displays the heart's rhythm and electrical impulses. The ECG helps in diagnosing various heart conditions, such as arrhythmias, heart attacks, and other cardiac abnormalities. The test involves attaching electrodes to the skin, and the resulting waveform provides valuable information about the heart's health and function.

Generally speaking, the ECG signal is a multidimensional signal composed of different leads, which are mandatory to describe the three-dimensional nature of the electrical propagation and distribution on the trunk surface.

A lead is usually described by either a monopolar or a bipolar electrode set, while a reference electrode is always considered. A general lead vector can be interpreted as a monitored spatial direction of cardiac electrical activity. In clinics, the standard ECG setup is described by 12 leads, with six of them called limb leads, and the other six called chest or precordial leads. The 12 leads are attained by at least ten electrodes, six on the chest surface, and 4 at the upper and lower limb extremities. In recent times, despite initial skepticism regarding its clinical significance, there has been a growing interest in the use of fewer leads even up to the so-called single-lead electrocardiogram (ECG) measurement. This surge in attention can be attributed to the widespread adoption of wearable devices, particularly smartwatches. These portable technologies offer a convenient means to monitor heart activity, potentially enhancing personal health awareness and early detection of cardiac irregularities. In the context of smartwatches, the acquisition of a single-lead measure involves the strategic placement of two differential electrodes. One is situated on the underside of the watch case, making contact with the wrist dorsum, while the other is positioned on the crown of the watch. To establish the necessary differential electrical connection, users are required to touch the crown with their fingertip, typically utilizing the right index finger when the watch is worn on the left hand. From an electrophysiological standpoint, with the arm registering a voltage roughly equivalent to the shoulder, this differential measure aligns with the standard lead I in the 12-lead ECG set. Termed an "on-demand" measure, it distinguishes itself from continuous measurements, necessitating intentional interaction from the user, who must touch the watch crown to activate the measurement. Operationally, the user waits for the completion of ECG recording process, usually taking just a few seconds to a minute. Head-mountable devices encompasses any type of device designed to be worn over at least one eye of a wearer and having a frame configured to be mounted on a head, including any eyewear.

In particular, smart head-mountable devices are known which comprise electronic devices adapted to operate as an electronic calculator capable of supporting the activities carried out by the user wearing them.

More in particular, the electronic devices usually comprise a processing and control unit and sensors integrated into the head-mountable devices.

For example, it is known to provide eyewear with an ECG detection systems for detecting an electrocardiogram signal in order to monitor the heartbeats of a user wearing the eyewear, for example during a sport training.

Such known ECG detection systems comprise a first electrode and a second electrode generally mounted on a first temple and on a second temple of a frame of an eyewear respectively; in details, the electrodes are mounted so as to face and contact the head of the user wearing the eyewear on opposite sides. In this way the ECG signal detected by the electrodes is due to the potential difference of two head opposite points. Moreover, thanks to this known ECG detection system the ECG signal can be continuously detected for all time period the user wears the eyewear.

US11298064B1, EP3213673A1 and US2023172468A1 shows some example of known eyewear with ECG detection systems.

Such known ECG detection systems can also be integrated in a generic head-mountable device in which the first and the second electrode are mounted so as to face and contact the head of the user wearing the head-mountable device on opposite sides.

However, the ECG signal so detected usually results to be weak and noisy since potentials differences recorded on the head resulting from heart electrical activity are in the order of microVolts and moreover the ECG signal can also be overlapped to electroencephalogram (EEG) signal. Therefore, the monitoring based on the ECG signal detected with the known detection system is low-amplitue and not sufficient reliable and accurate. Another drawback of the known ECG detection systems is that the ECG detection is made continuously and so the amount of data to be recorded is very high, thus requiring high storage space. This implies high costs. The object of the present invention is to overcome the above-mentioned drawbacks and in particular to devise a head-mountable device capable of detecting an ECG signal and an ECG detection system more reliable and accurate with respect to the prior art and which are cheaper than the prior art.

This and other objects according to the present invention are achieved by making a head-mountable device as set forth in claim 1 and an ECG detection system as defined in claim 14.

Further characteristics of the head-mountable device are the objects of the dependent claims.

The characteristics and advantages of the head-mountable device and of the ECG detection system according to the present invention will be more evident from the following exemplary though non-limiting description, referring to the attached schematic drawings in which:

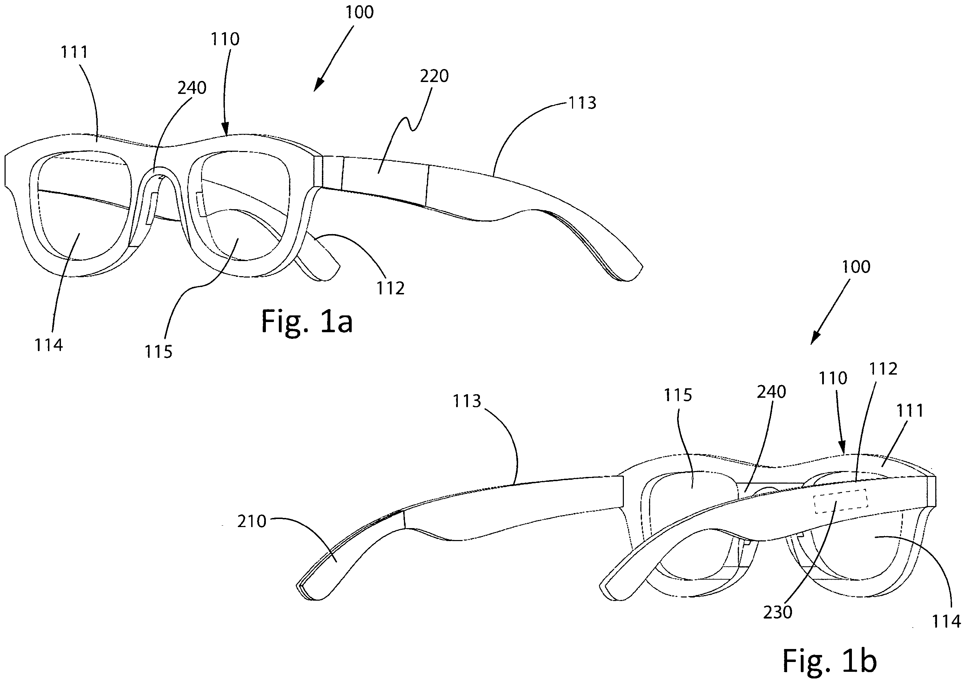

- Figures 1a and 1b are two schematic perspective views of a head-mountable device, in this cases an eyewear, according to the present invention;

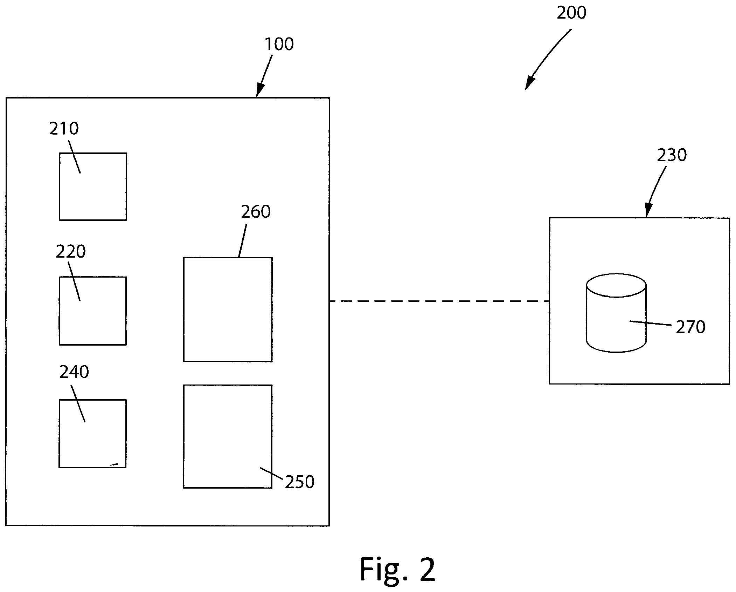

- Figure 2 is a block scheme of an embodiment of a head-mountable device according to the present invention as the one of figures 1a and 1b.

With reference to the figures, a head-mountable device is shown, globally referred to as 100.

For example, the head-mountable device may be a helmet, or a visor, or a mask or an eyewear and so on.

In particular, in the illustrated figures the head-mountable device 100 is an eyewear.

The head-mountable device 100 comprises a supporting structure 110 configured to be mounted on a head.

For example in the case of a helmet the supporting structure is the shell of the helmet, whereas in the case of the eyewear the supporting structure is the frame of the eyewear.

The head-mountable device 100 comprises also:
- at least one first electrode 210 arranged on the supporting structure 110 so as to be in contact with the head of a user when such a user wears the head-mountable device 100 in order to detect a first electrical potential;
- at least one second electrode 220 arranged on the supporting structure 110 so as to not be continuously in contact with a body part of a user when such a user wears the head-mountable device 100 in order to detect a second electrical potential on demand.

Advantageously, the head-mountable device 100 is configured to be connected to an electronic unit 230 configured to collect the electric potentials detected by the at least one first electrode 210 and the at least one second electrode 220 and to determine the electrocardiographic signal on the basis of the difference of said detected electric potentials.

In this description, with the term "electronic unit" it is intended to indicate an electronic processing and control unit, such as for example a microcontroller. The at least one first electrode 210 acts as the differential minus of the ECG lead to be recorded whereas the at least one second electrode 220 acts as the differential plus of the ECG lead to be recorded. Since the at least one second electrode 220 is intended to not be continuously in contact with a body part of the user, such at least one second electrode 220 does not continuously detect the second electric potential; the detection of the second electric potential occurs on demand when the user contacts the at least one second electrode 220 with a body part. Therefore, the ECG signal can be determined only when the user contacts the at least one second electrode 220 with a body part. This means that the determination of the ECG signal is not continuously but it can be done on demand of the user.

For example the body part with which the at least one second electrode 220 is intended to enter into contact is a finger.

The determination of the ECG signal, according to the present invention, is more accurate than the prior art since it is based on the larger potential difference between the head and the finger of a user and not on the lower potential difference between two points of the head.

The electronic unit 230 may be external to the head-mountable device 100, for example comprised in a personal computer, or in a laptop, or in mobile terminal like a smartphone or a smartwatch. In this case the head-mountable device 100 makes part of an ECG detection system 200 for detecting an electrocardiographic (ECG) signal that comprises the head-mountable device 100 and the electronic unit 230.

Alternatively, the electronic unit 230 may be integrated in the head-mountable device 100, in particular it may be associated to the structure 110. The electronic unit 230 can comprise an internal memory 270 that may be used also for locally storing the ECG signals determined. Moreover the electronic unit 230 may be configured to perform digital processing on the detected difference of the electric potentials so as to enhance signal quality and/or signal to noise ratio. For example such a digital processing may be the application of one or more digital filters.

Preferably, the at least one first electrode (210) is coupled to the supporting structure (110).

In this case the at least one first electrode 210 may be made as an electrically conducting plate attached to the structure 110, for example a plate made of metal or of a dry conducting polymer such as polyaniline or polypyrrole.

As an alternative the supporting structure 110 may comprise a first conducting portion made of electrically conducting material, thus the first conducting portion realizing the at least one first electrode 210.

In this case the first conducting portion may be made of metal or of a dry conducting polymer such as polyaniline or polypyrrole.

Preferably the at least one second electrode 220 is coupled to the supporting structure 110.

In this case the at least one second electrode 220 may be made as an electrically conducting plate attached to the structure 110, for example a plate made of metal or of a dry conducting polymer such as polyaniline or polypyrrole.

As an alternative the supporting structure 110 may comprise a second conducting portion made of electrically conducting material, thus the second conducting portion realizing the at least one second electrode 220.

In this case the second conducting portion may be made of metal or of a dry conducting polymer such as polyaniline or polypyrrole.

Preferably, the head-mountable device 100 is an eyewear and the supporting structure 110 is a frame 110 comprising a front 111 and two temples 112, 113 coupled to the front 111.

The eyewear may comprise also at least one lens 114, 115 coupled to the front 111. For example, the eyewear is an eyeglass and in this case it can comprise two lenses; the eyewear 100 can be a also a ski-mask or a mask sunglass and in this case it can comprise a single lens.

In this case preferably the at least one first electrode 210 is coupled with a respective temple 112, 113.

As an alternative at least one of said temples 112, 113 comprises the first conducting portion.

In any case, the at least one first electrode 210 may be positioned in the portion of the temple 112, 113 intended to be supported by the area that connects one ear to the head of the user, when such a user wears the eyewear 100. The at least one first electrode 210, in particular, faces towards the inner space enclosed by the frame 110.

Preferably the at least one second electrode 220 is arranged on the frame 110 on the opposite side of the at least one first electrode 210. In this case, if the at least one first electrode 210 faces towards the inner space enclosed by the frame 110, the at least one second electrode 220 faces towards the outer space. Preferably, the at least one second electrode 220 is coupled with a respective temple 112, 113.

As an alternative at least one of the temples 112, 113 comprises the second conducting portion.

Preferably, the eyewear 100 comprises a third electrode 240 arranged in a nose pad of the frame 110 so as to contact the nose of the user, when such a user wears the eyewear 110.

In this way the third electrode 240 acts as ground potential electrode. In this case, the electronic unit 230 is configured to determine the ECG signal also on the basis of the potential detected by the third electrode 240. This improves the accuracy of the ECG signal determined.

In case the electronic unit 230 is integrated in the head-mountable device 100, such a head-mountable device 100 may comprise also a battery 250 associated to the electronic unit 230 so as to electrically supply it. In case the electronic unit 230 is external with respect to the head-mountable device 100 the battery 250 is associated to the electronic components provided in the head-mountable device 100.

The head-mountable device 100 may also comprise a wireless communication module 260 configured to communicate in a wireless manner with an electronic terminal, such as a smartphone, thus allowing for convenient monitoring and analysis by healthcare professionals or personal devices.

In case the electronic unit 230 is integrated in the head-mountable device 100 the wireless communication module 260 is associated to the electronic unit 230.

In case the electronic unit 230 is external with respect to the head-mountable device 100 the wireless communication module 260 is configured to communicate in a wireless manner with such an electronic unit 230. In this case, the wireless communication module 260 may be electrically supplied by the battery 250, if present.

The head-mountable device 100 may comprise also a control interface to provide a start/stop control, real-time feedback on ECG measurements and system status, for example a push button or a touch interface. From the description made, the characteristics of the eyewear, object of the present invention, are clear, as are the relative advantages.

The eyewear, according to the present invention, is designed for on-demand single-lead electrocardiogram (ECG) monitoring, providing a convenient and user-friendly method for capturing cardiac data.

The invention is applicable in various scenarios, including fitness tracking, health monitoring, and early detection of cardiac abnormalities. The design allows for seamless integration into daily activities without compromising comfort or obstructing the user's field of view.

The ECG signals determined by the eyewear according to the present invention exhibits superior stability as compared to smartwatches, attributed to the inclusion of a ground/reference point. This additional reference enhances signal reliability and accuracy, ensuring more robust and consistent ECG recordings.

The eyewear according to the present invention may employ advanced dry polymer electrodes, a departure from conventional metal electrodes. This choice enhances user comfort by being more skin-tolerant, ensuring a gentle and irritation-free experience during prolonged contact.

Moreover, distinguishing itself from traditional leads that typically measure differentials on the arm or shoulder, our glasses implement a novel approach by capturing differentials on the head. This innovative configuration provides a unique perspective on ECG monitoring, contributing to a more comprehensive understanding of cardiac activity.

Finally, it is clear that the eyewear thus conceived is susceptible of numerous modifications and variations, all of which are within the scope of the invention; moreover, all the details may be replaced by technically equivalent elements. In practice, the materials used, as well as their dimensions, can be of any type according to the technical requirements.

## Claims

1. Head-mountable device (100) comprising:
- a supporting structure (110) configured to be mounted on a head of a user;
- at least one first electrode (210) arranged on the supporting structure (110) so as to be in contact with the head of a user when such a user wears the head-mountable device (100) in order to detect a first electrical potential;
- at least one second electrode (220) arranged on the supporting structure (110) so as to not be continuously in contact with a body part of a user when such a user wears the head-mountable device (100) in order to detect a second electrical potential on demand;
wherein said head-mountable device (100) is configured to be connected to an electronic unit (230) configured to collect the electric potentials detected by the at least one first electrode (210) and the at least one second electrode (220) and to determine the electrocardiographic signal on the basis of the difference of said detected electric potentials.

2. Head-mountable device (100) according to claim 1 wherein said electronic unit (230) is integrated in the head-mountable device (100).

3. Head-mountable device (100) according to claim 1 or 2 wherein the at least one first electrode (210) is coupled to the supporting structure (110).

4. Head-mountable device (100) according to claim 1 or 2 wherein the supporting structure (110) comprises a first conducting portion made of electrically conducting material, said first conducting portion realizing said at least one first electrode (210).

5. Head-mountable device (100) according to one or more of the preceding claims wherein the at least one second electrode (220) is coupled to the supporting structure (110) .

6. Head-mountable device (100) according to one or more of the claims from 1 to 4 wherein the supporting structure (110) comprises a second conducting portion made of electrically conducting material, said second conducting portion realizing said at least one second electrode (220).

7. Head-mountable device (100) according to one or more of the preceding claims wherein the head-mountable device (100) is an eyewear and the supporting structure (110) is a frame (110) comprising a front (111) and two temples (112, 113) coupled to the front (111).

8. Head-mountable device (100) according to claim 7 wherein the at least one first electrode (210) is coupled with a respective temple (112, 113).

9. Head-mountable device (100) according to claim 7 wherein at least one of said temples (112, 113) comprises the first conducting portion.

10. Head-mountable device (100) according to one or more of the claims from 7 to 9 wherein the at least one first electrode (210) is positioned in the portion of the temple intended to be supported by the area that connects one ear to the head of the user, when such a user wears the eyewear (100).

11. Head-mountable device (100) according to one or more of the claims from 7 to 10 wherein the at least one second electrode (220) is coupled with a respective temple (112, 113).

12. Head-mountable device (100) according to one or more of the claims from 7 to 10 wherein at least one of said temples (112, 113) comprises the second conducting portion.

13. Head-mountable device (100) according to one or more of the claims from 7 to 12 comprising a third electrode (240) arranged in a nose pad of the frame (110) so as to contact the nose of the user, when such a user wears the eyewear (100).

14. ECG detection system (200) for detecting an electrocardiographic (ECG) signal comprising:
- a head-mountable device (100) comprising:
- a supporting structure (110) configured to be mounted on a head;
- at least one first electrode (210) arranged on the supporting structure (110) so as to be in contact with the head of a user when such a user wears the head-mountable device (100) for detecting a first electrical potential;
- at least one second electrode (220) arranged on the supporting structure (110) so as to not be in contact with a body part of a user when such a user wears the head-mountable device (100) for detecting a second electrical potential on demand;
- an electronic unit (230) configured to be connected to the head-mountable device (100) and to collect the electric potentials detected by the at least one first electrode (210) and the at least one second electrode (220) and to determine the electrocardiographic signal on the basis of the difference of said detected electric potentials.
